# FASCICULE DE BREVET EUROPEEN

(11) **EP 4 057 998 B1**
(45) Date de publication et mention de la délivrance du brevet: **13.05.2026**
(21) Numéro de dépôt: 20823891.5
(22) Date de dépôt: 13.11.2020
(51) Int. Cl.: A61K 9/50, A61K 31/606, A61P 1/04

(54) **PROCÉDÉ DE FABRICATION D'UNE COMPOSITION PHARMACEUTIQUE À ADMINISTRATION ORALE ET À DÉLIVRANCE COLONIQUE**
PROZESS ZUR HERSTELLUNG EINER PHARMAZEUTISCHEN ZUSAMMENSETZUNG ZUR ORALEN EINNAHME UND WIRKSTOFFFREISETZUNG IM KOLON
PROCESS FOR MANUFACTURING A PHARMACEUTICAL COMPOSITION FOR ORAL INTAKE AND DRUG RELEASE IN THE COLON

(30) Priorité: 13.11.2019 FR 1912643; 13.11.2019 CN 201911103911
(43) Date de publication de la demande: 21.09.2022
(73) Titulaire: Ethypharm, 92210 Saint-Cloud (FR); Shanghai Ethypharm Pharmaceuticals Co. Ltd., 200003 Shanghai (CN)
(72) Inventeur: HERRY, Catherine, 27670 Saint-Ouen du Tilleul (FR); ZHU, Yuqiu, Shanghai (CN); CRIERE, Bruno, 27930 Gravigny (FR)
(74) Mandataire: Regimbeau
(86) Numéro de dépôt international: PCT/FR2020/052082
(87) Numéro de publication internationale: WO 2021/094697

(56) Documents cités:
- EP-A1- 0 366 621
- EP-A1- 2 659 881

## Description

### DOMAINE DE L'INVENTION

La présente invention porte sur un procédé de préparation d'une composition pharmaceutique à administration orale et à délivrance colonique comprenant au moins un cœur et une couche d'enrobage.

### ART ANTERIEUR

La mésalazine ou acide 5-aminosalicylique est un anti-inflammatoire communément utilisé pour le traitement des maladies inflammatoires du côlon telles que la colite ulcérative ou la maladie de Crohn.

La mésalazine agit localement au niveau du côlon. Or, l'administration orale de mésalazine est rendue problématique par le fait que ce médicament est presque complètement absorbé dans l'intestin grêle et que, par conséquent, seule une petite quantité atteint le côlon pour assurer son action thérapeutique.

Dans le passé, dans le but de surmonter cette difficulté, des formulations de mésalazine avec des revêtements particuliers ont été développées, présentant la caractéristique de ne libérer le principe actif que dans la zone souhaitée, ainsi que pour éviter les effets secondaires systémiques. Ces compositions pharmaceutiques sont des formes à libération retardée ou lente, appropriées pour empêcher ou retarder l'absorption de la mésalazine dans le tractus proximal afin d'obtenir des concentrations thérapeutiques dans l'iléon et le côlon. Par exemple, la demande de brevet européen EP 0 040 590 (demandeur Aktiebolaget Hässle) décrit des préparations pharmaceutiques orales capables de libérer un médicament, par exemple la mésalazine, sélectivement dans le côlon, à un pH supérieur à 5,5. Ceci est obtenu par enrobage d'un cœur contenant le principe actif par un mélange d'un polymère acrylique anionique soluble juste à pH 5,5, tel que, par exemple, l'Eudragit L, en quantités allant de 10 à 85%, et d'un polymère acrylique substitué par de l'ammonium quaternaire, insoluble dans l'eau, comme par exemple Eudragit RS ou RL, en quantités allant de 15 à 90%. Ces compositions sont fabriquées d'abord par fabrication du cœur par mélange du principe actif avec un matériau de charge puis extrusion/sphéronisation ou par dépôt du principe actif à la surface d'une particule de support ou par fabrication d'un cœur ne contenant que le principe actif ; puis, le cœur est revêtu et cette application du revêtement est réalisée de préférence grâce à un appareil à lit fluidisé. Cependant, les compositions décrites dans ce document ne permettent pas d'obtenir un enrobage homogène et reproductible, ce qui entraine une dissolution de la composition et une libération de l'actif non homogène dans le tractus intestinal. En effet, la fabrication de microgranules par extrusion/sphéronisation conduit à l'obtention de microgranules hétérogènes en taille ; ainsi, lors de l'enrobage, ces microgranules recevront une quantité de polymère différente suivant leur taille ce qui formera une couche polymérique hétérogène. L'impact de cette hétérogénéité de couche polymérique se traduit par la suite par une variabilité de dissolution entre les microgranules revêtus.

Des alternatives ont ensuite été recherchées, visant à agir sur le temps de transit de la bouche à l'iléon terminal de la composition pharmaceutique fabriquée ou sur l'activité enzymatique de la flore microbienne colonique.

Néanmoins, il existe toujours un besoin de disposer d'un procédé de fabrication d'une composition pharmaceutique à administration orale et libération colonique permettant que la libération du principe actif de la composition obtenue soit homogène et ciblée dans le côlon et qui soit simple, reproductible et économique.

### RESUME DE L'INVENTION

Dans le cadre de la présente invention, les inventeurs ont découvert un procédé de préparation d'une composition pharmaceutique permettant de libérer un principe actif, notamment la mésalazine, dans le côlon, de manière spécifique et homogène. Ce procédé implique une technique de poudrage du principe actif qui permet de garantir une granulométrie et un enrobage homogènes et reproductibles avec de faibles coefficients de variation, et donc une libération du principe actif également homogène et reproductible avec de faibles coefficients de variation.

La présente invention porte ainsi sur un procédé de préparation d'une composition pharmaceutique à administration orale et à délivrance colonique comprenant au moins un cœur et une couche d'enrobage, caractérisé en ce qu'il comprend les étapes suivantes :
a) Pulvérisation sur un support neutre d'une suspension aqueuse comprenant au moins un copolymère (méth)acrylate anionique soluble à pH supérieur à 5,5 et au moins un principe actif destiné à être délivré dans le côlon ;
   ou
a') Pulvérisation sur un support neutre d'une suspension aqueuse comprenant au moins un copolymère (méth)acrylate anionique soluble à pH supérieur à 5,5 puis
b') Poudrage d'au moins un principe actif destiné à être délivré dans le côlon sur les microgranules obtenus après l'étape a') ;
c') réalisation des étapes a') et b') alternativement jusqu'à obtention de la teneur en principe actif voulue
   et
d) Enrobage des microgranules obtenus après l'étape a) ou c') par pulvérisation d'une composition comprenant au moins un copolymère (méth)acrylate anionique soluble à pH supérieur à 6, un copolymère (méth)acrylate anionique soluble à pH supérieur à 7 et un copolymère (méth)acrylate anionique insoluble en milieu aqueux.

### DESCRIPTION DETAILLEE DE L'INVENTION

### Définitions

Par « délivrance colonique » ou « libération colonique » on entend, au sens de la présente invention, la libération sélective d'un principe actif au niveau du côlon, également appelé gros intestin.

Par libération ou dissolution « homogène » on entend, au sens de la présente invention, une dissolution homogène entre les microgranules d'une composition pharmaceutique, en termes de temps de dissolution et de la zone du tractus intestinal dans laquelle ils libèrent le principe actif qu'ils renferment. Une dissolution homogène vise à obtenir une même cinétique de libération du principe actif dans le corps entre les différents microgranules. Dans le cadre de la présente invention, la dissolution homogène vise à permettre une libération de principe actif localisée au niveau du côlon par la majorité des microgranules de la composition selon l'invention, ceci permettant d'une part une plus grande concentration en principe actif au niveau de la zone cible thérapeutique qu'est le côlon et d'autre part d'éviter les effets secondaires liés à la libération de principe actif dans les autres régions du tractus intestinal et à l'absorption du principe actif au niveau de l'intestin grêle.

Par libération ou dissolution « spécifique » ou « ciblée » on entend, au sens de la présente invention le fait que la libération du principe actif est spécifique ou ciblée sur la partie de l'intestin considérée par rapport aux autres régions du tractus intestinal.

Par « copolymère anionique », on entend, au sens de la présente invention, un copolymère contenant des groupes anioniques.

Par « copolymère (méth)acrylate » on entend, au sens de la présente invention, un copolymère obtenu par polymérisation de monomères (méth)acrylates tels que l'acide acrylique, l'acide méthacrylique ou leurs esters. Le copolymère (méth)acrylate peut notamment être un polymère issu de la polymérisation d'au moins deux des monomères suivants : l'acide acrylique, l'acide méthacrylique, l'acrylate d'éthyle, le méthacrylate de méthyle, et l'ester d'acide méthacrylique avec groupement ammonium quaternaire.

Par « copolymère insoluble dans l'eau » on entend, au sens de la présente invention, tout copolymère insoluble dans l'eau ou dans une solution physiologique.

Par « D50 » on entend, au sens de la présente invention, le diamètre présenté par 50% de la population de microgranules considérée.

Par « D90 » on entend, au sens de la présente invention, le diamètre présenté par 90% de la population de microgranules considérée.

### Procédé de préparation d'une composition pharmaceutique selon l'invention

La présente invention porte sur un procédé de préparation d'une composition pharmaceutique à administration orale et à délivrance colonique comprenant au moins un cœur et une couche d'enrobage, caractérisé en ce qu'il comprend les étapes suivantes :
a) Pulvérisation sur un support neutre d'une suspension aqueuse comprenant au moins un copolymère (méth)acrylate anionique soluble à pH supérieur à 5,5 et au moins un principe actif destiné à être délivré dans le côlon ;
   ou
a') Pulvérisation sur un support neutre d'une suspension aqueuse comprenant au moins un copolymère (méth)acrylate anionique soluble à pH supérieur à 5,5 puis b') Poudrage d'au moins un principe actif destiné à être délivré dans le côlon sur les microgranules obtenus après l'étape a') ;
c') réalisation des étapes a') et b') alternativement jusqu'à obtention de la teneur en principe actif voulue
   et
d) Enrobage des microgranules obtenus après l'étape a) ou c') par pulvérisation d'une composition comprenant au moins un copolymère (méth)acrylate anionique soluble à pH supérieur à 6, un copolymère (méth)acrylate anionique soluble à pH supérieur à 7 et un copolymère (méth)acrylate anionique insoluble en milieu aqueux.

Ce procédé permet donc l'obtention d'une composition pharmaceutique à administration orale et à délivrance colonique comprenant au moins un cœur et une couche d'enrobage, ledit cœur comprenant au moins un support neutre, un principe actif destiné à être délivré dans le côlon et au moins un copolymère (méth)acrylate anionique soluble à pH supérieur à 5,5, ladite couche d'enrobage comprenant au moins un copolymère (méth)acrylate anionique soluble à pH supérieur à 6, un copolymère (méth)acrylate anionique soluble à pH supérieur à 7 et un copolymère (méth)acrylate anionique insoluble dans l'eau.

Selon un mode de réalisation préféré, la composition pharmaceutique à administration orale et à délivrance colonique obtenue à l'issue du procédé selon l'invention est sous forme de microgranules.

Selon un mode de réalisation préféré, le copolymère (méth)acrylate anionique soluble à pH supérieur à 5,5 de l'étape a) ou a') est un copolymère acide méthacrylique - acrylate d'éthyle (1:1). De préférence, ledit copolymère acide méthacrylique - acrylate d'éthyle (1:1) utilisé est le composé commercialisé Eudragit^{®} L30D. Le choix de ce copolymère comme liant tient à son intérêt dans la technique du poudrage.

Selon un mode de réalisation préféré, parmi les copolymères de l'étape d) ledit copolymère (méth)acrylate anionique soluble à pH supérieur à 6 est un copolymère acide méthacrylique - méthyl-méthacrylate (1:1). De préférence, ledit copolymère acide méthacrylique - méthyl-méthacrylate (1:1) utilisé est le composé commercialisé Eudragit^{®} L100. Ce copolymère permet de retarder la libération dans le tractus intestinal des composés enrobés par ce copolymère jusqu'au niveau du jéjunum.

Selon un mode de réalisation préféré, parmi les copolymères de l'étape d) ledit copolymère (méth)acrylate anionique soluble à pH supérieur à 7 est un copolymère acide méthacrylique - méthyl-méthacrylate (1:2). De préférence, ledit copolymère acide méthacrylique - méthyl-méthacrylate (1:2) utilisé est le composé commercialisé Eudragit^{®} S100. Ce copolymère permet de retarder la libération dans le tractus intestinal des composés enrobés par ce copolymère jusqu'au niveau de l'iléon et du côlon.

Selon un mode de réalisation préféré, parmi les copolymères de l'étape d) ledit copolymère insoluble en milieu aqueux est un copolymère acrylate d'éthyle - méthyl-méthacrylate - ester d'acide méthacrylique avec groupement ammonium quaternaire (1:2:0,2), avantageusement un copolymère acrylate d'éthyle - méthyl-méthacrylate - ester d'acide méthacrylique avec groupement chlorure de méthacrylate de triméthylammonioéthyle (1:2:0,2). De préférence, ledit copolymère acrylate d'éthyle - méthyl-méthacrylate - ester d'acide méthacrylique avec groupement ammonium quaternaire utilisé est le composé commercialisé Eudragit^{®} RL100. Ce copolymère permet de contrôler dans le temps la libération des composés enrobés par ce copolymère.

Selon un mode de réalisation préféré, la composition pulvérisée à l'étape d) présente un ratio copolymère (méth)acrylate anionique soluble à pH supérieur à 6: copolymère (méth)acrylate anionique soluble à pH supérieur à 7 : copolymère (méth)acrylate anionique insoluble en milieu aqueux de 4:3:3. Le mélange des trois copolymères de l'enrobage permet d'obtenir une libération de la composition pharmaceutique qui diffère en fonction du pH car ces trois polymères se solubilisent à des pH différents. Ce mélange des trois polymères permet ainsi la génération d'aspérités de l'enrobage à mesure que la composition pharmaceutique se déplace dans le transit intestinal et la libération du produit à l'endroit souhaité, c'est-à-dire dans le côlon.

Dans le cadre de la présente invention, les trois polymères de l'enrobage sont appliqués en une même couche. Ceci diffère des enrobages de l'art antérieur dans lesquels chaque polymère forme une couche distincte. Ainsi, la composition pharmaceutique préparée selon le procédé selon l'invention, peut comprendre une ou plusieurs couche(s) d'enrobage, chacune des couches comprenant le mélange des trois polymères cités ci-dessus.

Avantageusement, la composition pharmaceutique préparée selon le procédé de l'invention, comprend une seule couche d'enrobage.

La préparation d'une composition pharmaceutique avec un enrobage comprenant tous les polymères dans une seule couche et avantageusement comprenant une monocouche d'enrobage, permet notamment un gain de temps important et donc un gain économique. En outre, l'enrobage monocouche permet un meilleur ajustement du taux d'enrobage, notamment en cas de problème technique lors de sa préparation (problème air comprimé, bouchage buse, ou autre). En effet, lorsque l'enrobage est multicouche, chacune des couches comprenant un polymère différent, un ajustement de l'enrobage et notamment des premières couches d'enrobage ne pourra pas être effectué, ce qui est problématique, notamment si un problème technique survient lors de l'application des premières couches. En effet, les problèmes techniques ne sont détectés qu'à la fin du procédé, c'est à dire lorsque toutes les couches ont été appliquées, il ne sera donc pas possible de réajuster l'enrobage des premières couches.

Selon un mode de réalisation préféré, le poudrage du principe actif à l'étape b') est réalisé par poudrage manuel ou mécanique dans au moins une turbine conventionnelle à fond plat. Ce procédé de poudrage manuel ou mécanique permet d'obtenir et de garantir, de manière avantageuse, des cœurs de la composition pharmaceutique sous la forme de microgranules présentant une distribution granulométrique resserrée.

Cette granulométrie resserrée permet par la suite de réaliser un enrobage homogène et reproductible autour des microgranules ; en effet, comme les microgranules présentent tous une taille équivalente grâce au poudrage manuel ou mécanique, ils recevront la même quantité de polymère qui formera une couche d'épaisseur équivalente sur tous les microgranules. Lors de l'administration de la composition pharmaceutique, cela permettra l'obtention de faibles coefficients de variation lors de la dissolution des granules dans le corps, c'est-à-dire une dissolution homogène entre les microgranules enrobés de la composition pharmaceutique.

Avantageusement et de manière non limitative, le principe actif destiné à être délivré dans le côlon peut être un anti-infectieux, par exemple un antibiotique, un agent anti-inflammatoire, anti-histaminique, anti-cholinergique, antiviral, antimitotique, des peptides, des protéines, des gènes, des oligonucléotides anti-sens, des agents de diagnostic et/ou des agents immunosuppressifs ou des bactéries.

Avantageusement, le principe actif destiné à être délivré dans le côlon peut être hydrosoluble ou liposoluble.

Parmi les principes actifs destinés à être délivrés dans le côlon particulièrement avantageux, on trouve les agents anti-inflammatoires, les agents antitumoraux, les oligonucléotides anti-sens et les enzymes capables d'inactiver les antibiotiques au niveau du côlon, notamment les β-lactamases ou les enzymes capables d'inactiver les macrolides et apparentés comme l'érythromycine estérase.

Plus avantageusement, le principe actif destiné à être délivré dans le côlon est un agent anti-inflammatoire.

De préférence, le principe actif destiné à être délivré dans le côlon est choisi parmi la salicylazosulfapyridine ou sulfasalazine (Salazopyrine), l'acide 5-aminosalicylique (mesalazine), le budesonide, la rifamycine, l'acamprosate ou le linaclotide.

### FIGURES

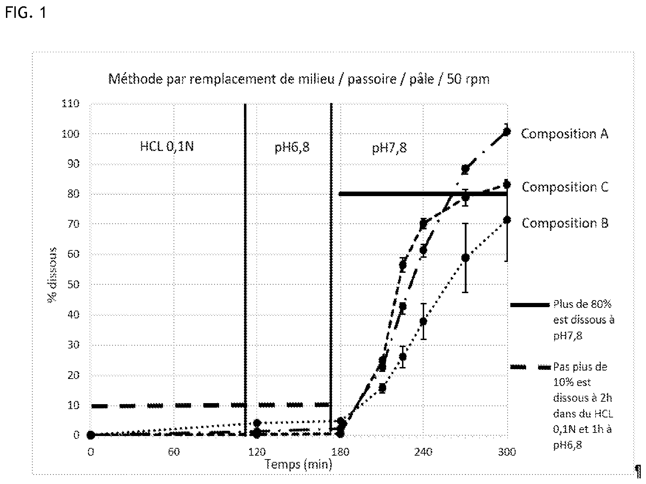

FIG. 1 : Elle décrit les résultats de l'exemple 3.

Les exemples qui suivent visent à illustrer la présente invention de manière non limitative.

### EXEMPLES

### Exemple 1 : Préparation d'une composition pharmaceutique selon l'invention par poudrage en turbine conventionnelle

La composition A décrite dans le Tableau 2 a été préparée selon le protocole suivant :
1) Montage des microgranules de Mesalazine :
   - Introduction des supports neutres dans la turbine (Température de masse ; 15 - 35°C) ;
   - Pulvérisation de la suspension liante comprenant l'Eudragit L30D et le triéthylcitrate ;
   - Poudrage du principe actif par poudrage manuel ou mécanique ;
   - Séchage ; et
   - Tamisage sur un tamis vibrant de type SODEVA avec des grilles de 500 à 1000 µm.

A la fin du montage, les microgranules présentent un D50 compris entre 500 et 700 µm et un D90 compris entre 700 et 950 µm.

### 2) Enrobage

Suite à l'étape de montage, les microgranules sont enrobés en pulvérisant la suspension d'enrobage sur les microgranules actifs.

Composition de la suspension d'enrobage pour un gain de poids de 10% en polymère pour une pulvérisation sur 1000.0 g de grains actifs de Mesalazine :
- Eudragit S100 : 30.0g
- Eudragit RL100 : 30.0g
- Eudragit L100 : 40.0g
- TEC (triéthylcitrate) : 15.0g
- Talc :40.0g
- Ethanol : 1395.0g

La suspension d'enrobage est préparée selon le protocole suivant :
- Peser l'Ethanol dans un récipient de capacité adaptée et le mettre sous agitation avec un agitateur (type IKA) équipé d'une hélice.
- Incorporer sous agitation l'Eudragit^{®} S100 et attendre la solubilisation complète.
- Incorporer sous agitation L'Eudragit^{®} L100 et attendre la solubilisation complète.
- Incorporer sous agitation l'Eudragit^{®} RL100 et attendre la solubilisation complète.
- Ajouter le Triéthyl Citrate sous agitation et maintenir l'agitation pendant 1 h.
- Incorporer le Talc sous agitation et attendre 30 minutes avant de démarrer la pulvérisation.
- Pulvériser la suspension d'enrobage sur les microgranules en maintenant l'agitation durant toute la pulvérisation, tel que décrit ci-après.

L'enrobage est réalisé dans un lit d'air fluidisé type Glatt GPCG1 en mode Wurster équipé d'un pistolet Schlick (buse 1.2mm).

Paramètres d'enrobage :

**Tableau 1**

| Paramètres | Consigne |
|---|---|
| Température d'entrée d'air | 30-35°C |
| Température produit | 25-35°C |
| Débit d'air | 40-60 m3/h |
| Débit de pulvérisation suspension | 3-10 g/min |
| Air nébulisation | 1-2 bar |

A l'issue de l'enrobage les microgranules sont tamisés sur une grille de taille adaptée.

A la fin de l'enrobage, les microgranules présentent un D50 compris entre 600 et 800 µm et un D90 compris entre 800 et 950 µm.

**Tableau 2**

| | | | |
|---|---|---|---|
| Composition A selon l'invention | | Sachets de 1000mg | Sachets de 2000mg |
| Ingrédient pharmaceutique actif | Mesalazine | 1000 mg | 2000 mg |
| Supports neutres | Neutres SP 400-500 µm | 594.4 mg | 1188.8 mg |
| copolymère (méth)acrylate anionique soluble à pH supérieur à 5,5 | Eudragit^{®} L30D | 74.9 mg | 149.7 mg |
| Copolymère (méth)acrylate anionique soluble à pH supérieur à 6 | Eudragit^{®} L100 | 67.1 mg | 134.1 mg |
| Copolymère (méth)acrylate anionique soluble à pH supérieur à 7 | Eudragit^{®} S100 | 50.3 mg | 100.6 mg |
| Copolymère (méth)acrylate anionique insoluble dans l'eau | Eudragit^{®} RL100 | 50.3 mg | 100.6 mg |
| Plastifiant | Triéthyl Citrate | 25.2 mg | 50.3 mg |
| Lubrifiant | Talc | 67.1 mg | 134.1 mg |
| Total | | 1936.6 mg | 3873.2 mg |

### Exemple 2 comparatif : Préparation des compositions comparatives B et C par poudrage en turbine conventionnelle

Les compositions comparatives B et C décrites dans les Tableaux 3 et 4 ont été préparées suivant le même protocole que celui décrit dans l'exemple 1.

**Tableau 3**

| Composition comparative B | | |
|---|---|---|
| Cœur | Mesalazine | 52,8 % |
| | Neutre SP 400-500 µm | 31,4 % |
| | Eudragit^{®} L30D | 4,0 % |
| | Triéthylcitrate | 0,4 % |
| Enrobage 1 | Eudragit^{®} E100 | 2,7 % |
| | Triéthylcitrate | 0.3 % |
| | Talc | 1.3 % |
| Enrobage 2 | Eudragit^{®} S100 | 4,6 % |
| | Triéthylcitrate | 0.7% |
| | Talc | 1.8% |
| | | 100,0 % |

**Tableau 4**

| Composition comparative C | | |
|---|---|---|
| Cœur | Mesalazine | 51,6 % |
| | Neutre SP 400-500 µm | 30,7 % |
| | Eudragit^{®} L30D | 3,9 % |
| | Triéthylcitrate | 0,4 % |
| Enrobage | Eudragit^{®} S100 | 8,7 % |
| | Triéthylcitrate | 1,3 % |
| | Talc | 3,4 % |
| TOTAL | | 100,0 % |

### Exemple 3 : Etude du profil de dissolution des compositions A, B et C

Protocole : l'appareil utilisé est un dissolutest de type USP II (agitation par pâles). L'équivalent de 1 g de principe actif sous forme de microgranules est introduit dans un vase contenant 750 mL de HCl 0.1N, mis en agitation pendant 2 heures à la vitesse de 50 rpm. Un prélèvement de 10 mL est effectué au bout des 2 heures. Les grains sont récupérés et introduits dans un vase contenant 950 mL d'un milieu tampon de pH 6.8 (composé de 6.805 g de KH2PO4 et 22.4 mL de NaOH pour 1 L) mis en agitation pendant 1 heure à la vitesse de 50 rpm. Un prélèvement de 10 mL est effectué au bout de 1 heure. 50 mL d'une solution NaOH 0.36N sont ajoutés dans le vase. L'agitation est activée à 50 rpm. Des prélèvements de 5 mL sont effectués au bout de 30, 45, 60, 90 et 120 min.

Les prélèvements sont ensuite analysés par chromatographie liquide et la quantité de principe actif libérée est déterminée par détection UV.

Résultats : les résultats sont présentés dans la figure 1, qui illustre les pourcentages de dissolution des compositions A, B et C en fonction du temps.

Conclusion : le profil de dissolution de la composition A est : <10% après 2h en HCl 0.1N et 1h en pH6.8, puis > 80% après 2h en pH7.8.

Les inventeurs ont ainsi mis en évidence que le procédé selon l'invention permet d'obtenir une composition pharmaceutique présentant une dissolution homogène et reproductible et donc une libération du principe actif également homogène et reproductible avec de faibles coefficients de variation.

## Revendications

1. Procédé de préparation d'une composition pharmaceutique à administration orale et à délivrance colonique comprenant au moins un cœur et une couche d'enrobage, **caractérisé en ce qu'**il comprend les étapes suivantes :
a) Pulvérisation sur un support neutre d'une suspension aqueuse comprenant au moins un copolymère (méth)acrylate anionique soluble à pH supérieur à 5,5 et au moins un principe actif destiné à être délivré dans le côlon ;
ou
a') Pulvérisation sur un support neutre d'une suspension aqueuse comprenant au moins un copolymère (méth)acrylate anionique soluble à pH supérieur à 5,5 puis b') Poudrage d'au moins un principe actif destiné à être délivré dans le côlon sur les microgranules obtenus après l'étape a') ;
c') réalisation des étapes a') et b') alternativement jusqu'à obtention de la teneur en principe actif voulue
et
d) Enrobage des microgranules obtenus après l'étape a) ou c') par pulvérisation d'une composition comprenant au moins un copolymère (méth)acrylate anionique soluble à pH supérieur à 6, un copolymère (méth)acrylate anionique soluble à pH supérieur à 7 et un copolymère (méth)acrylate anionique insoluble en milieu aqueux.

2. Procédé selon la revendication 1, **caractérisé en ce que** ledit copolymère (méth)acrylate anionique soluble à pH supérieur à 5,5 de l'étape a) ou a') est un copolymère acide méthacrylique - acrylate d'éthyle (1:1).

3. Procédé selon la revendications 1 ou 2, **caractérisé en ce que** parmi les copolymères de l'étape d) ledit copolymère (méth)acrylate anionique soluble à pH supérieur à 6 est un copolymère acide méthacrylique - méthyl-méthacrylate (1:1).

4. Procédé selon l'une quelconque des revendications 1 à 3, **caractérisé en ce que** parmi les copolymères de l'étape d) ledit copolymère (méth)acrylate anionique soluble à pH supérieur à 7 est un copolymère acide méthacrylique - méthyl-méthacrylate (1:2).

5. Procédé selon l'une quelconque des revendications 1 à 4, **caractérisé en ce que** parmi les copolymères de l'étape d) ledit copolymère insoluble en milieu aqueux est un copolymère acrylate d'éthyle - méthyl-méthacrylate - ester d'acide méthacrylique avec groupement ammonium quaternaire (1:2:0,2), avantageusement un copolymère acrylate d'éthyle - méthyl-méthacrylate - ester d'acide méthacrylique avec groupement chlorure de méthacrylate de triméthylammonioéthyle (1:2:0,2).

6. Procédé selon l'une quelconque des revendications 1 à 5, **caractérisé en ce que** la composition pulvérisée à l'étape d) présente un ratio copolymère (méth)acrylate anionique soluble à pH supérieur à 6 : copolymère (méth)acrylate anionique soluble à pH supérieur à 7 : copolymère (méth)acrylate anionique insoluble en milieu aqueux de 4:3:3.

7. Procédé selon l'une quelconque des revendications 1 à 6, **caractérisé en ce que** ledit principe actif destiné à être délivré dans le côlon est choisi parmi la sulfasalazine, l'acide 5-aminosalicylique (mesalazine), le budesonide, la rifamycine, l'acamprosate ou le linaclotide.

8. Procédé selon l'une quelconque des revendications 1 à 7, **caractérisé en ce que** la composition pharmaceutique à administration orale et à délivrance colonique obtenue à l'issue du procédé est sous forme de microgranules.

9. Procédé selon l'une quelconque des revendications 1 à 8, **caractérisé en ce que** le poudrage du principe actif à l'étape b') est réalisé par poudrage manuel ou mécanique dans au moins une turbine conventionnelle.

## Patentansprüche

1. Verfahren zur Herstellung einer pharmazeutischen Zusammensetzung zur oralen Verabreichung und zur Freisetzung im Kolon, die mindestens einen Kern und eine Umhüllungsschicht umfasst, **dadurch gekennzeichnet, dass** es die folgenden Schritte umfasst:
a) Zerstäuben, auf einem neutralen Träger, einer wässrigen Suspension, die mindestens ein lösliches anionisches (Meth)acrylat-Copolymer mit einem pH-Wert von mehr als 5,5 und mindestens einen Wirkstoff umfasst, der dazu bestimmt ist, im Kolon freigesetzt zu werden;
oder
a') Zerstäuben, auf einem neutralen Träger, einer wässrigen Suspension, die mindestens ein lösliches anionisches (Meth)acrylat-Copolymer mit einem pH-Wert von mehr als 5,5 umfasst, dann
b') Bepudern des nach dem Schritt a') erhaltenen Mikrogranulats mit mindestens einem Wirkstoff, der dazu bestimmt ist, im Kolon freigesetzt zu werden;
c') abwechselndes Ausführen der Schritte a') und b') bis zum Erhalten des gewünschten Gehalts an Wirkstoff
und
d) Umhüllen des nach dem Schritt a) oder c') erhaltenen Mikrogranulats mittels Zerstäubens einer Zusammensetzung, die mindestens ein lösliches anionisches (Meth)acrylat-Copolymer mit einem pH-Wert von mehr als 6, ein lösliches anionisches (Meth)acrylat-Copolymer mit einem pH-Wert von mehr als 7 und ein nicht in einem wässrigen Medium lösliches anionisches (Meth)acrylat-Copolymer umfasst.

2. Verfahren nach Anspruch 1, **dadurch gekennzeichnet, dass** das lösliche anionische (Meth)acrylat-Copolymer mit einem pH-Wert von mehr als 5,5 des Schritts a) oder a') ein Methacrylsäure-Ethylacrylat-Copolymer (1:1) ist.

3. Verfahren nach Anspruch 1 oder 2, **dadurch gekennzeichnet, dass** unter den Copolymeren des Schritts d) das lösliche anionische (Meth)acrylat-Copolymer mit einem pH-Wert von mehr als 6 ein Methacrylsäure-Methylmethacrylat-Copolymer (1:1) ist.

4. Verfahren nach einem der Ansprüche 1 bis 3, **dadurch gekennzeichnet, dass** unter den Copolymeren des Schritts d) das lösliche anionische (Meth)acrylat-Copolymer mit einem pH-Wert von mehr als 7 ein Methacrylsäure-Methylmethacrylat-Copolymer (1:2) ist.

5. Verfahren nach einem der Ansprüche 1 bis 4, **dadurch gekennzeichnet, dass** unter den Copolymeren des Schritts d) das nicht in einem wässrigen Medium lösliche Copolymer ein Ethylacrylat-Methylmethacrylat-Methacrylsäureester-Copolymer mit quaternärer Ammoniumgruppe (1:2:0,2), vorzugsweise ein Ethylenacrylat-Methylmethacrylat-Methacrylsäureester mit Trimethylammoniumethylmethacrylatchloridgruppe (1:2:0,2) ist.

6. Verfahren nach einem der Ansprüche 1 bis 5, **dadurch gekennzeichnet, dass** die im Schritt d) zerstäubte Zusammensetzung ein Verhältnis zwischen löslichem anionischem (Meth)acrylat-Copolymer mit einem pH-Wert von mehr als 6 : löslichem anionischen (Meth)acrylat-Copolymer mit einem pH-Wert von mehr als 7 : nicht in einem wässrigen Medium löslichem anionischem (Meth)acrylat-Copolymer 4:3:3 beträgt.

7. Verfahren nach einem der Ansprüche 1 bis 6, **dadurch gekennzeichnet, dass** der Wirkstoff, der dazu bestimmt ist, im Kolon freigesetzt zu werden, ausgewählt wird aus Sulfasalazin, 5-Aminosalicylsäure (Mesalazin), Budesonid, Rifamycin, Acamprosat oder Linaclotid.

8. Verfahren nach einem der Ansprüche 1 bis 7, **dadurch gekennzeichnet, dass** die pharmazeutische Zusammensetzung zur oralen Verabreichung und zur Freigabe im Kolon, die aus dem Verfahren erhalten wird, in der Form von Mikrogranulat vorliegt.

9. Verfahren nach einem der Ansprüche 1 bis 8, **dadurch gekennzeichnet, dass** das Bepudern mit dem Wirkstoff im Schritt b') durch manuelles oder mechanisches Bepudern in mindestens einer herkömmlichen rotierenden Trommel ausgeführt wird.

## Claims

1. A process for preparing an orally administered pharmaceutical composition with colonic delivery comprising at least one core and a coating layer, **characterized in that** it comprises the following steps:
a) Spraying onto a neutral support an aqueous suspension comprising at least one anionic (meth)acrylate copolymer that is soluble at a pH greater than 5.5 and at least one active ingredient intended to be delivered in the colon;
or
a') Spraying onto a neutral support an aqueous suspension comprising at least one anionic (meth)acrylate copolymer that is soluble at a pH greater than 5.5 then
b') Dusting at least one active ingredient intended to be delivered in the colon onto the microgranules obtained after step a');
c') carrying out steps a') and b') alternately until the desired content of active ingredient has been obtained
and
d) Coating the microgranules obtained after step a) or c') by spraying a composition comprising at least one anionic (meth)acrylate copolymer that is soluble at a pH greater than 6, an anionic (meth)acrylate copolymer that is soluble at a pH greater than 7 and an anionic (meth)acrylate copolymer that is insoluble in an aqueous medium.

2. The process according to claim 1, **characterized in that** said anionic (meth)acrylate copolymer that is soluble at a pH greater than 5.5 of step a) or a') is a methacrylic acid-ethyl acrylate (1:1) copolymer.

3. The process according to claim 1 or 2, **characterized in that** among the copolymers of step d) said anionic (meth)acrylate copolymer that is soluble at a pH greater than 6 is a methacrylic acid-methyl methacrylate (1:1) copolymer.

4. The process according to any one of claims 1 to 3, **characterized in that** among the copolymers of step d) said anionic (meth)acrylate copolymer that is soluble at a pH greater than 7 is a methacrylic acid-methyl methacrylate (1:2) copolymer.

5. The process according to any one of claims 1 to 4, **characterized in that** among the copolymers of step d) said copolymer that is insoluble in an aqueous medium is an ethyl acrylate-methyl methacrylate-methacrylic acid ester with a quaternary ammonium group copolymer (1:2:0.2), advantageously an ethyl acrylate-methyl methacrylate-methacrylic acid ester with a trimethylammonioethyl methacrylate chloride group copolymer (1:2:0.2).

6. The process according to any one of claims 1 to 5, **characterized in that** the composition sprayed in step d) has a ratio anionic (meth)acrylate copolymer that is soluble at a pH greater than 6: anionic (meth)acrylate copolymer that is soluble at a pH greater than 7: anionic (meth)acrylate copolymer that is insoluble in an aqueous medium of 4:3:3.

7. The process according to any one of claims 1 to 6, **characterized in that** said active ingredient intended to be delivered in the colon is selected from sulfasalazine, 5-aminosalicylic acid (mesalazine), budesonide, rifamycin, acamprosate or linaclotide.

8. The process according to any one of claims 1 to 7, **characterized in that** the orally administered pharmaceutical composition with colonic delivery obtained at the end of the process is in the form of microgranules.

9. The process according to any one of claims 1 to 8, **characterized in that** the dusting of the active ingredient in step b') is carried out by manual or mechanical dusting in at least one conventional turbine.
